# EUROPÄISCHE PATENTSCHRIFT

(11) **EP 0 573 888 B1**
(45) Veröffentlichungstag und Bekanntmachung des Hinweises auf die Patenterteilung: **21.08.1996**
(21) Anmeldenummer: 93108849.6
(22) Anmeldetag: 02.06.1993
(51) Int. Cl.: A61F 2/20

(54) **Stimmprothese**
Voice prosthesis
Prothèse vocale

(30) Priorität: 06.06.1992 DE 4218739
(43) Veröffentlichungstag der Anmeldung: 15.12.1993
(73) Patentinhaber: ESKA Implants GmbH & Co., 23556 Lübeck (DE)
(72) Erfinder: Gundei, Hans, W-2400 Lübeck (DE)
(74) Vertreter: Fuchs, Luderschmidt & Partner Patentanwälte

(56) Entgegenhaltungen:
- DE-A- 3 211 126

## Beschreibung

Die vorliegende Erfindung betrifft eine Stimmprothese für laryngektomierten Patienten, d. h. Patienten, deren Kehlkopf operativ entfernt worden ist und die dadurch ihre natürliche Stimme verloren haben.

Früher erfolgte eine stimmliche Rehabilitation von Laryngektomierten entweder durch das Erlernen der sogenannten Oesophagusstimme, zu deren Erzeugung der Patient Luft in seine Speiseröhre (Oesophagus) preßte und durch bewußte Entspannung der Pseudoglottis den Luftstrom freigeben konnte, welcher durch seine Zunge und Lippen einer Artikulation unterworfen wurde. Eine weitere Möglichkeit bestand in der Verwendung sogenannter Servox-Geräte, die an den Halsbereich gesetzt wurden und welche vibrierten. Die Vibrationsschwingungen wurden einer Artikulation durch die Bewegung der Lippen unterzogen.

Das Klangbild dieser so erzeugten Ersatzstimmen entspricht in keiner Weise einem natürlichen Klangbild. Es werden sehr tiefe und "knarrende" Ersatzstimmen erzeugt. Wohl aus diesem Grunde ist die Verwendung dieser Ersatzstimmen insbesondere für weibliche Patienten mit einer extrem hohen psychologischen Hemmschwelle behaftet.

Anfang der 80er Jahre konnte operativ insofern eine Verbesserung hervorgerufen werden, als daß ein Shunt zwischen der ansonsten im Rachenbereich operativ verschlossenen Luftrohre (Trachea) und dem Oesophagus hergestellt werden konnte. In diesen Shunt wurden sogenannte Stimmprothesen eingesetzt. Atmen konnte der Patient durch die operativ hergestellte Verbindung zwischen der Trachea und der Umwelt durch das sogenannte Tracheostoma, welches auch bei den früheren Operationstechniken hergestellt werden mußte und in neuerer Zeit durch sogenannte Tracheostomaventile, wie es beispielsweise in der EP-B-0 221 973 beschrieben ist, geschützt wird. Wollte der Patient nun sprechen, so verschloß er das Tracheostoma mit einem Finger oder das Ventil schloß aufgrund des höheren Luftdruckes selbsttätig, so daß Luft aus der Trachea durch den Shunt in den Oesophagus strömen konnte. Eine derartige sogenannte Stimmprothese ist beispielsweise bekannt aus der DD-275183 A1. Die Bezeichnung des darin beschriebenen Einwegeventils als Stimmprothese ist freilich unzutreffend. Dieses Ventil leistet nämlich nichts anderes, als einen Luftstrom zwischen der Trachea und dem Oesophagus zuzulassen und ein Eindringen von Speisepartikeln oder Speichel vom Oesophagus zurück in die Trachea zu verhindern. Letzteres ist zwar extrem wichtig, da ansonsten Komplikationen wie Lungenentzündungen etc. auftreten könnten. Technisch gesprochen aber erschließt das in der besagten Druckschrift beschriebene Ventil nur eine andere Luftquelle im Vergleich zu jener bei der Erzeugung der weiter oben beschriebenen Oesophagusstimme. Die mit den Ventil erzeugte Ersatzstimme ist ebenfalls extrem tief in ihrer Frequenzlage und daher insbesondere für weibliche Patienten schwer akzeptabel.

Eine sogenannte Kehlkopfprothese gemäß dem Oberbegriff des Anspruchs 1 zeigt die DE-A-32 11 126. Diese Prothese bildet nicht anderes als einen Übergang zwischen Trachea und dem Oesophagus, wie bereits weiter beschrieben. Ähnliche Prothesen zeigen die US-A-4,808,183, die EP-A-0 279 485 und die EP-A-O 222 509.

Einen anderen Weg zeigt beispielsweise die DE-A-22 53 496 und die JP-A-2174843. Darin sind Vorrichtungen vorgesehen, bei denen extrakorporal ein tonerzeugendes Element angeordnet ist, um eine künstlich erzeugte Frequenz zur Sprachmodulation heranzuziehen. Die Anordnung als extrakorporale Vorrichtung ist freilich für den Patienten sehr belastend, u.a. da diese Vorrichtungen für jeden Gesprächspartner sichtbar sind.

Vor diesem Hintergrund ist es nun die Aufgabe der vorliegenden Erfindung, eine Stimmprothese zum Einsatz in den Shunt zwischen Trachea und Oesophagus eines laryngektomierten Patienten anzugeben, mit welcher die Frequenzlage der Ersatzstimme der Frequenzlage der natürlichen Stimme angepaßt werden kann, wobei sämtliche Teile der Stimmprothese intrakorporal implantierbar sein sollen.

Gelöst wird die Aufgabe durch die Stimmprothese mit dem kennzeichnenden Merkmal des Anspruchs 1. Verschiedene Ausführungsformen sind in den Unteransprüchen angegeben.

Demgemäß ist vorgesehen, daß bei einer Stimmprothese, die aus einem röhrenförmigen Metallteil besteht, welches zur trachealen Seite hin eine offene trichterförmige Aufweitung aufweist, innerhalb des Strömungsweges der Luft von der Trachea in den Oesophagus zumindest ein durch den durch die Prothese hindurchtretenden Luftstrom in Schwingung versetzbares und dadurch einen hörbaren Ton erzeugendes Element vorgesehen ist. Im Gegensatz zu den herkömmlichen sogenannten Stimmprothesen, die - wie bereits ausgeführt - nichts anderes als ein Einwegeventil darstellen, weist die erfindungsgemäße Stimmprothese ein schwingfähiges Element auf, welches mit einer voreingestellten Frequenz schwingen kann. Dieses Element versetzt die Luftsäule innerhalb der Stimmprothese in Schwingungen, welche daraufhin in den Oesophagus und Rachenraum des Patienten weitergeleitet werden. Die Artikulation von Worten geschieht wiederum durch die Lippenbewegung. Durch den Einsatz des schwingfähigen Elementes ist es möglich, bestimmte Stimmlagen für jeden Patienten individuell voreinzustellen durch geeignete Auswahl des schwingfähigen Elementes. So wird man für weibliche Patienten ein schwingfähiges Element auswählen, welches einen höheren Ton generiert als ein Element, welches bei männlichen Patienten zum Einsatz kommen wird.

Wie ein herkömmliches Shunt-Ventil verbleibt die erfindungsgemäße Stimmprothese bis ca. 8 Wochen in situ. Dann erfordern Ablagerungen und Pilzbefall (Candida) den Wechsel und die Reinigung der Prothese. Diese stellt aber im wesentlichen kein Problem dar. Hierzu wird die Stimmprothese wie ein herkömmliches Shuntventil durch das Tracheostoma entfernt und eine neue oder die gereinigte Stimmprothese wird durch das Tracheostoma hindurch in den Shunt zwischen Trachea und Oesophagus gesetzt.

Die Stimmprothese verharrt in situ dadurch, daß das röhrenförmige Metallteil in bekannter Weise zwei ringförmige Flansche beispielsweise aus Silikon trägt, zwischen denen die z. B. zusammengenähten Wände des Oesophagus und der Trachea zu liegen kommen.

Gemäß einer ersten Ausführungsform der Stimmprothese ist vorgesehen, daß das den Ton erzeugende Element eine die trichterförmige Aufweitung bedeckende flexible Membran ist, welche den Rand der trichterförmigen Aufweitung hintergreift und zumindest einen Schlitz im Bereich der durch sie bedeckten Aufweitung aufweist.

Beim Verschließen des Tracheostomas wird die flexible Membran angeblasen und dadurch in Schwingung versetzt. Der zumindest eine Schlitz in der Membran erzeugt einen Ton, bildlich gesprochen wie die zusammengepreßten Lippen eines Trompetenspielers. Das Hintergreifen des Randes der trichterförmigen Aufweitung durch die Membran sorgt für einen außerordentlich stabilen Sitz der Membran bei gleichzeitiger Möglichkeit der freien Schwingungen ihrer übrigen Teile.

Vorzugsweise ist die trichterförmige Aufweitung so dimensioniert, daß die Membran den Boden der Aufweitung nicht berührt, selbst wenn sie mit maximaler Schwingungsamplitude schwingt. Durch diese Maßnahme wird erreicht, daß auch bei hohem Luftstrom und damit bei hohem Anpreßdruck an der Membran die künstliche Stimme nicht einfach "wegbleibt".

Die Membran besteht vorzugsweise aus Silikonkautschuk mit einer Shore-Härte im Bereich von 30 bis 70. Eine hohe Shore-Härte resultiert in einer relativ hohen Schwingfrequenz, eine niedrige Shore-Härte hingegen in einer relativ tiefen Frequenz. Die Stimmprothese kann durch Wahl des Membranmaterials patientenindividuell konfektioniert werden. So werden in aller Regel weibliche Patienten eine Membran mit einer hohen Shore-Härte erhalten, männliche Patienten hingegen eine mit relativ niedriger Shore-Härte.

Gemäß einer anderen Ausführungsform der erfindungsgemäßen Stimmprothese ist das den Ton erzeugende Element ein im freien Lumen des röhrenförmigen Metallteils in dessen Wandung verankertes metallisches Stimmplättchen. Das Stimmplättchen weist eine im wesentlichen L-förmige Gestalt auf, wobei der kurze Schenkel zur Verankerung dient, der längere hingegen die vorgesehene Schwingung ausführt. Die Stimmprothese gemäß dieser Ausführungsform läßt sich sehr genau konfektionieren in Hinblick auf die durch sie erzeugte Schwingungsfrequenz, nämlich durch Auswahl des geeigneten Stimmplättchens. Die Schwingfrequenz des Stimmplättchen hängt in erster Linie ab von der Breite seines schwingenden Schenkels, von der Materialdicke sowie von der Länge des schwingenden Schenkels. Diese Parameter können sehr genau eingestellt werden. Dem jeweiligen Patienten kann dann eine Stimmprothese an die Hand gegeben werden, die über eine patientenindividuell ausgewählte Schwingfrequenz verfügt.

Ein besonderes Augenmerk muß bei der erfindungsgemäßen Stimmprothese darauf gelegt werden, daß keinerlei Speisepartikel oder Speichel in sie eindringen können, da sie ansonsten ihre Funktion nicht mehr ausüben könnte. Gemäß einer vorteilhaften Weiterbildung weisen alle Ausführungsformen der Stimmprothese mindestens oesophagusseitig eine Schutzvorrichtung auf, welche vor dem Eintreten von Speisepartikeln und Speichel schützt.

Gemäß einer ersten Ausführungsform ist vorgesehen, daß das röhrenförmige Metallteil der Stimmprothese oesophagusseitig durch ein Einwegeventil verschließbar ist. Vorteilhaft ist das Ventil gebildet durch eine im Inneren des röhrenförmigen Metallteils bewegbare Kugel und Öffnungen in der Seitenwandung des röhrenförmigen Metallteils, wobei die Kugel zwischen einer den Durchgang zur trichterförmigen Aufweitung freigebenden und eine diesen verschließenden Stellung bewegbar ist. Setzt der Patient zum Sprechen an, so leitet er willkürlich einen höheren Luftstrom durch die erfindungsgemäße Stimmprothese. Der erhöhte Luftstrom drückt die erwähnte Kugel so weit vor sich her, bis die Öffnungen freigegeben werden, so daß Luft aus ihnen heraustreten kann in den Oesophagus.

Die Schutzwirkung kann noch dadurch erhöht werden, daß die Öffnungen in der Seitenwandung durch Schutzlaschen vor dem Eintreten von Fremdkörper in die Trachea geschützt sind. Diese Schutzlaschen können aus elastischem Silikon bestehen und mit dem röhrenförmigen Metallteil oberhalb der Öffnungen verklebt sein. Die Schutzlaschen überdecken die Öffnungen wie ein schützendes Dach.

Gemäß einer anderen Ausführungsform ist das oesophagusseitige Einwegeventil ein Flatterventil, gebildet aus einem über das röhrenförmige Metallteil geschobenen Kunststoffschlauch, welcher am oesophagusseitigen Ende bis auf einen Längsschlitz verschlossen ist. Hebt der Patient zum Sprechen an, so spreizt der erhöhte Luftstrom den Kunststoffschlauch im Bereich des Längsschlitzes etwas auf, so daß die Luft aus der Stimmprothese austreten kann. Nach dem Beenden des Sprechvorgangs schließt sich der Längsschlitz von selbst durch die materialimmanenten Rückstellkräfte.

Gemäß einer anderen Ausführungsform ist die oesophagusseitige Schutzvorrichtung kein Einwegeventil. Es kann vielmehr vorgesehen sein, daß das röhrenförmige Metallteil der Stimmprothese oesophagusseitig wiederum an seiner Stirnöffnung verschlossen ist und in diesem Bereich in der Seitenwandung mindestens eine Seitenöffnung vorgesehen ist. Diese ist durch eine schützende, den aus der Seitenöffnung austretenden Luftstrom umleitende Abdeckhaube überdeckt.

In vorteilhafter Weiterbildung ist zusätzlich zur oesophagusseitigen Schutzvorrichtung tracheaseitig eine weitere Schutzvorrichtung in Form eines Einwege-Kugelventils vorgesehen. Diese Maßnahme erhöht die Schutzwirkung gegen Verschmutzung des Inneren der Stimmprothese erheblich, wodurch Beeinträchtigungen des Schwingelementes Membran oder Stimmplättchen wirksam unterbunden werden. Ein positiver Nebeneffekt des zusätzlichen tracheaseitigen Kugelventils ist darin zu sehen, daß der Patient aufgrund dieser Anordnung wieder vermehrt durch den Rachenraum atmen kann anstelle durch sein Tracheostoma.

In konkreter Ausbildung kann die tracheaseitige Schutzvorrichtung so ausgebildet sein, daß im Inneren des röhrenförmigen Metallteils im tracheaseitigen Bereich eine Kugel freibeweglich gelagert ist, die in ihrer Bewegung in Richtung fort von der trichterförmigen Aufweitung, also in Richtung Oesophagus, begrenzt ist durch einen das röhrenförmige Metallteil diametral durchsetzenden Anschlag und in der Bewegung in Richtung der trichterförmigen Aufweitung begrenzt ist durch einen in diesem ausgeformten Kugelventilsitz. Der das röhrenförmige Metallteil durchsetzende Anschlag kann beispielsweise ein Metallplättchen oder ein Draht sein. Vorzugsweise bietet er einen nur geringen Strömungswiderstand gegen die durch die Stimmprothese strömende Luft. Hebt der Patient zum Sprechen an, wird die Kugel gegen den besagten Anschlag gedrückt werden und ein Luftstrom durch die Stimmprothese wird ermöglicht. Beim Einatmen durch das Tracheostoma hingegen wird die Kugel in den Kugelventilsitz an der Rückseite der trichterförmigen Aufweitung gesogen aufgrund des dann an der Lufteintrittsöffnung der Stimmprothese vorherrschenden Unterdrucks.

Eine weitere Ausführungsform der Stimmprothese mit einer zusätzlichen tracheaseitigen Schutzvorrichtung in Form eines Einwege-Kugelventils ist so ausgebildet, daß die tracheaseitige Schutzvorrichtung in einem länglichen röhrenförmigen Aufsatzstück ausgebildet ist, welches auf die trichterförmige Aufweitung aufgeschoben ist, die trichterförmige Aufweitung hintergreift und in seinem Inneren die Kugel des Einwege-Kugelventils freibeweglich lagert, die in ihrer Bewegung in Richtung auf die trichterförmige Aufweitung durch einen ihr Lumen diametral durchsetzenden Anschlag und in ihrer Bewegung in Richtung auf die Lufteintrittsöffnung durch einen in ihrem Inneren ausgebildeten Kugelventilsitz begrenzt ist. Der Anschlag sowie der Kugelventilsitz ist im Prinzip so ausgebildet wie in dem vorbeschriebenen Ausführungsbeispiel. In vorteilhafter Weise sind an dem röhrenförmigen Aufsatzstück die bereits angesprochenen ringförmigen Flansche angeformt, zwischen denen die zusammengefaßten Oesophagus- und Tracheawand liegen. Vorzugsweise besteht das Aufsatzstück aus Silikon.

Die Stimmprothese gemaß der Erfindung ist vorteilhaft in etwa L-förmig ausgebildet, so daß das oesophagusseitige Ende nach dem Einsetzen der Stimmprothese in den Shunt zwischen Trachea und Oesophagus kranialwärts, d.h. nach oben weist.

Die Erfindung wird anhand einiger Ausführungsbeispiele näher erläutert. Hierbei zeigt:
Fig. 1 ein erstes Ausführungsbeispiel der Stimmprothese,
Fig. 2 ein zweites Ausführungsbeispiel,
Fig. 3 ein drittes Ausführungsbeispiel,
Fig. 4 ein viertes Ausführungsbeispiel,
Fig. 5 ein fünftes Ausführungsbeispiel,
Fig. 6 ein sechstes Ausführungsbeispiel,
und Figuren 7 (a-c) Aufsichten auf unterschiedliche Ausführungsformen der Membran.

In den Zeichnungsfiguren sind sich entsprechende Teile mit denselben Bezugszeichen versehen.

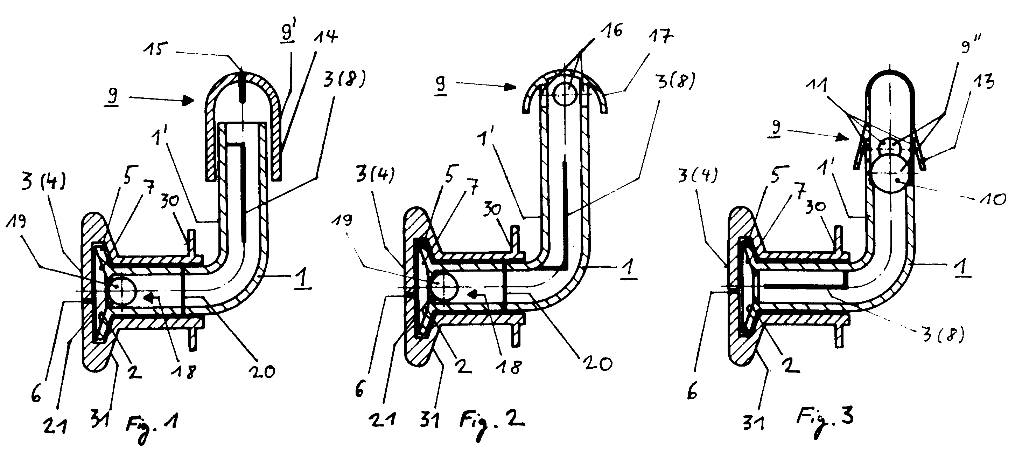

Fig. 1 zeigt ein erstes Ausführungsbeispiel für die erfindungsgemäße Stimmprothese. Kernstück der Stimmprothese ist das hier L-förmig gebogene, röhrenförmige Metallteil 1. An seinem in situ zur trachealen Seite liegenden Ende weist das Metallteil 1 eine offene trichterförmige Aufweitung 2 auf. Diese offene trichterförmige Aufweitung 2 ist in dem dargestellten Ausführungsbeispiel bedeckt durch ein beim Anblasen mit Luft einen hörbaren Ton erzeugenden Element 3, vorliegend durch eine Membran 4. Diese Membran 4 hintergreift den Rand 5 der trichterförmigen Aufweitung 2. Hierdurch wird ein außerordentlich stabiler Sitz der Membran 4 über der trichterförmigen Aufweitung 2 erreicht. Im dargestellten Ausführungsbeispiel ist das Membranteil rückseitig noch schlauchförmig verlängert dargestellt und trägt an seinem distalen Ende einen ringförmigen Flansch 30. Zwischen diesem Flansch 30 und der Rückseite 31 an der trichterförmigen Aufweitung 2 kommen die z. B. zusammengenähten Wände der Trachea und des Oesophagus in abdichtender Anlage zu liegen. Das die Membran 4 umfassende Membranteil mit seiner schlauchartigen Verlängerung und dem angeformten Flansch 30 besteht aus Silikonkautschuk. Die Shore-Härte dieses Materials liegt im Bereich von 30 bis 70. Eine höhere Shore-Härte läßt die Membran 4 einen höheren Ton, eine niedrigere Shore-Härte einen niedrigeren Ton erzeugen, wenn diese von der Trachea her mit Luft angeblasen wird. In der Membran 4 ist zur Tonerzeugung mindestens ein Schlitz 6 eingebracht, durch welchen die Luft in das röhrenförmige Metallteil 1 strömen kann. Die trichterförmige Aufweitung 2 ist so dimensioniert, daß die Membran 4 den Boden 7 der Aufweitung 2 nicht berührt, selbst wenn die Membran mit einer maximalen Schwingungsamplitude schwingt.

Die in Figur 1 dargestellte Stimmprothese weist darüber hinaus ein weiteres in Schwingung versetzbares und dadurch einen hörbaren Ton erzeugendes Element 3 auf, nämlich ein im freien Lumen des röhrenförmigen Metallteils 1 verankertes metallisches Stimmplättchen 8. Dieses Stimmplättchen 8 ist praktisch L-förmig gestaltet mit einem langen Schenkel, der im Lumen des Metallteils 1 schwingen kann, und mit einem kurzen Schenkel, der in der Wandung 1' des Metallteils 1 verankert ist, beispielsweise in einem darin vorgesehenen Schlitz.

Die Stimmprothese ist oesophagusseitig mit einer Schutzvorrichtung 9 versehen, die ein Eintreten von Speisepartikeln und Speichel in die Stimmprothese verhindert. Vorliegend ist das röhrenförmige Metallteil 1 mit einem Einwegeventil 9' versehen, welches aus einem über das röhrenförmige Metallteil 1 geschobenen Kunststoffschlauch 14 besteht. Dieser liegt eng an der Wandung 1' des röhrenförmigen Metallteils an, was aus Darstellungsgründen in Fig. 1 nicht erkennbar ist. Dieser Kunststoffschlauch weist an seinem oesophagusseitigen Ende einen Längsschlitz 15 auf. Strömt nun Luft von der Trachea zum Oesophagus, so wird sich der Längsschlitz spreizen und einen Luftstrom in den Rachenraum des Patienten zulassen. Wird der Luftstrom unterbrochen, etwa weil der Patient aufhört zu sprechen, so schließt sich der Längsschlitz aufgrund materialimmanenter Rückstellkräfte und verhindert wirksam ein Eindringen von Speisepartikeln oder Speichel in die Stimmprothese.

Die dargestellte Stimmprothese weist darüber hinaus als zusätzliche Schutzmaßnahme tracheaseitig eine Schutzvorrichtung in Form eines Einwege-Kugelventils 18 auf. Dieses besteht aus einer in dem röhrenförmigen Metallteil 1 gelagerten Kugel 19, die dort frei beweglich ist zwischen einem Anschlag 20 und einem Ventilsitz 21. Dieser Anschlag 20 kann ein in die Wandung 1' des röhrenförmigen Metallteils 1 eingelassener Metallstreifen oder -draht sein. In jedem Falle bietet er nur einen geringen Strömungswiderstand für den Luftstrom. Der Anschlag 20 begrenzt die Bewegung der Kugel 19 in Richtung fort von der trichterförmigen Aufweitung 2. In dem Mündungsbereich des freien Lumens des röhrenförmigen Metallteils 1 in die trichterförmige Aufweitung 2 ist ein Kugelventilsitz 21 ausgebildet, der eine weitere Bewegung nach links in der Zeichnung verhindert. Hebt der Patient nun zum Sprechen an, wird die Kugel 19 von der dargestellten Lage am Ventilsitz 21 hin zum Anschlag 20 gedrückt und Luft kann durch das röhrenförmige Metallteil 1 unter Anregung der Membran 4 und des Stimmplättchen 8 zum Schwingen und unter Aufweitung des Schlitzes 15 des Flatterventils von der Trachea in den Oesophagus strömen. Die in den Rachenraum gelangenden Schwingungen werden sodann vom Patienten einer Artikulation unterworfen.

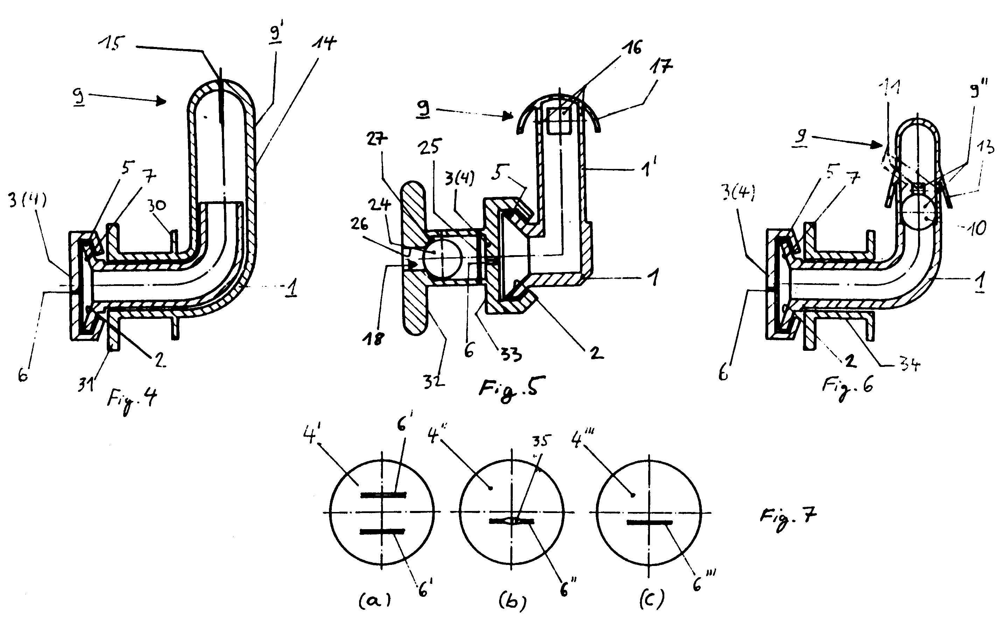

Fig. 2 zeigt ein zweites Ausführungsbeispiel. An dieser Stelle sei lediglich auf die Unterschiede zu der Stimmprothese gemäß Fig. 1 hingewiesen. Der Ort der Verankerung des Stimmplättchens 8 ist ein anderer als in der Fig. 1 dargestellt. Grundsätzlich kommt es auf den Verankerungsort des Stimmplättchen nicht an. Relevant hingegen ist, daß der lange, schwingende Schenkel genügend Raum hat, um auch Schwingungen mit maximaler Amplitude ausführen zu können.

Vorliegend unterscheidet sich die oesophagusseitige Schutzvorrichtung 9 von jener gemäß Fig. 1. Hier ist das röhrenförmige Metallteil 1 an seiner Stirnöffnung verschlossen. Anstelle dessen sind in der Seitenwandung 1' Seitenöffnungen 16 vorgesehen, die durch eine sie überdeckende Abdeckhaube 17 geschützt sind vor einem Eindringen von Speisepartikeln und Speichel. Die Abdeckhaube 17 kann integraler Bestandteil des röhrenförmigen Metallteils 1 sein. Sie kann aber auch mit dem Metallteil 1 verklebt sein und aus Silikon bestehen.

In Fig. 3 ist eine Ausführungsform der Stimmprothese gezeigt, welche im Gegensatz zu jenen aus Figuren 1 und 2 keine zusätzliche tracheaseitige Schutzvorrichtung aufweist. An dieser Stelle hingegen ist das Stimmplättchen 8 verankert. Auch die oesophagusseitige Schutzvorrichtung 9 unterscheidet sich von den vorstehend erläuterten Ausführungsformen. Hier nämlich ist das röhrenförmige Metallteil 1 oesophagusseitig wiederum geschlossen. Als Luftaustrittsöffnungen sind Öffnungen 11 in der Seitenwandung 1' des Metallröhrchens 1 vorgesehen. Im Inneren des röhrenförmigen Metallteils 1 ist eine Kugel 10 gelagert. Die Anordnung ist derart, daß, wenn der Patient zum Sprechen anhebt, die Kugel 10 durch den Luftstrom soweit angehoben wird, daß ein Durchgang zwischen der trichterförmigen Aufweitung 2 und den Öffnungen 11 freigegeben wird. Die Öffnungen 11 sind durch Schutzlaschen 13 überdeckt, die aus elastischem Material bestehen und oberhalb der Öffnungen 11 an dem Metallteil 1 beispielsweise angeklebt sind.

In Fig. 4 ist eine andere Ausführungsform der Stimmprothese dargestellt, die lediglich über ein einziges in Schwingung versetzbares und einen hörbaren Ton erzeugendes Element 3 verfügt, nämlich über die Membran 4, die den Rand 5 der trichterförmigen Aufweitung 2 hintergreift. Als oesophagusseitige Schutzvorrichtung 9 ist hier ein Einwege-Ventil 9' vorgesehen, das gebildet ist aus einem über das röhrenförmige Metallteil 1 geschobenen Kunststoffschlauch 14, das - wie bereits in Figur 1 gezeigt - über einen Längsschlitz 15 verfügt. Die Funktionsweisen sind entsprechend.

Im Unterschied zu jener Ausführungsform ist der Kunststoffschlauch 14 verlängert und weist als integrale Bestandteile die Flansche 30 und 31 auf, zwischen denen die Trachea- und Oesophaguswand in abdichtende Anlage kommen.

Figur 5 zeigt eine weitere Ausführungsform der Stimmprothese. Kernstück bildet hier wieder das röhrenförmige Metallteil 1 mit einer Schutzvorrichtung 9 am oesophagusseitigen Ende, die bereits anhand des Ausführungsbeispiels gemäß Fig. 2 erläutert worden ist. Als den Ton erzeugendes Element 3 ist hier wiederum eine Membran 4 vorgesehen, die in beschriebener Weise den Rand 5 der trichterförmigen Aufweitung 2 des röhrenförmigen Metallteils 1 hintergreift und die trichterförmige Aufweitung bedeckt. Sie ist integraler Bestandteil eines länglichen, röhrenförmigen Aufsatzstückes 22, welches in seinem röhrenförmigen Abschnitt als zusätzliche tracheaseitige Schutzvorrichtung ein Einwege-Kugelventil 18 beinhaltet, zu dem die bewegliche Kugel 24 gehört, die in ihrer Bewegung in Richtung auf die trichterförmige Aufweitung 2 duch einen den Rohrabschnitt durchsetzenden Anschlag 25 begrenzt ist. Dieser Anschlag ist ausgebildet wie der Anschlag 20, wie er anhand der Figuren 1 und 2 erläutert worden ist. Die Kugel 24 ist in ihrer Bewegung in Richtung auf die Lufteintrittsöffnung 26 begrenzt durch einen Kugelventilsitz 27, der im Mündungsbereich des Lumens des Rohrabschnittes in die Lufteintrittsöffnung 26 ausgebildet ist. Trachea- und Oesophaguswand kommen zwischen den als Flansche wirkenden Absätze 32 und 33 zur Anlage.

Ein letztes Ausführungsbeispiel zeigt die Fig. 6 mit wiederum mit nur einem einen Ton erzeugenden Element 3, nämlich einer Membran 4, die über die trichterförmige Aufweitung 2 gespannt ist und deren Rand 5 hintergreift. Die oesophagusseitige Schutzvorrichtung 9 entspricht jener, wie sie anhand von Fig. 3 bereits beschrieben worden ist. Im Unterschied zu den vorbeschriebenen Ausführungsbeispielen ist vorliegend ein muffenartiges Rohrstück 34, vorzugsweise aus Silikon, über das röhrenförmige Metallteil 1 geschoben bishin zu trichterförmigen Aufweitung 2. Das Teil 34 weist die beiden nicht näher bezeichneten Flansche auf, zwischen denen Trachea- und Oesophaguswand zur Anlage kommen.

In Fig. 7 schließlich sind drei Aufsichten auf Membranen 4', 4'' und 4''' dargestellt. Die Membran 4' gemäß Fig. 7a weist zwei Schlitze 6' auf. Die Membran gamäß 7b weist lediglich einen Schlitz 6'', welcher etwa in seiner Mitte eine lochartige Aufweitung 35 aufweist. Die Membran 4''' gemäß Fig. 7c weist hingegen lediglich einen Schlitz 6''' auf. Mit den unterschiedlichen Anzahlen von Schlitzen bzw. deren Ausbildung lassen sich Parameter wie die Tonhöhe beeinflussen.

## Patentansprüche

1. Stimmprothese zum Einsatz in den Shunt zwischen Trachea und Oesophagus eines laryngektomierten Patienten, bestehend aus einem röhrenförmigen Metallteil (1) mit einer zur trachealen Seite hin offenen trichterförmigen Aufweitung (2), dadurch gekennzeichnet, daß innerhalb des Luftströmungsweges der Prothese von der Trachea in den Oesophagus mindestens ein durch den durch sie hindurchtretenden Luftstrom in Schwingung versetzbares und dadurch einen hörbaren Ton erzeugendes Element (3) sitzt.

2. Stimmprothese nach Anspruch 1, bei der das den Ton erzeugende Element eine die trichterförmige Aufweitung (2) bedeckende flexible Membran (4,4',4'',4''') ist, welche den Rand (5) der trichterförmigen Aufweitung (2) hintergreift und zumindest einen Schlitz (6,6',6'',6''') im Bereich der durch sie bedeckten Aufweitung (2) aufweist.

3. Stimmprothese nach Anspruch 1, bei der die trichterförmige Aufweitung (2) so dimensioniert ist, daß die Membran (4,4',4'',4''') den Boden (7) der Aufweitung (2)selbst bei der maximalen Schwingungsamplitude nicht berührt.

4. Stimmprothese nach Anspruch 2 oder 3, bei der die Membran (4,4',4'',4''') aus Silikonkautschuk besteht mit einer Shore-Härte im Bereich von 30 bis 70.

5. Stimmprothese nach Anspruch 1, bei der das den Ton erzeugende Element ein im freien Lumen des röhrenförmigen Metallteils (1) in dessen Wandung (1') verankertes metallisches Stimmplättchen (8) ist.

6. Stimmprothese nach einem der Ansprüche 1 bis 5, die mindestens oesophagusseitig durch eine Schutzvorrichtung (9) vor dem Eintreten von Speisepartikeln und Speichel geschützt ist.

7. Stimmprothese nach Anspruch 6, bei der das röhrenförmige Metallteil (1) oesophagusseitig durch ein Einwegeventil (9',9'') verschließbar ist.

8. Stimmprothese nach Anspruch 7, bei der das Ventil gebildet ist durch eine im Inneren des röhrenförmigen Metallteils (1) bewegbare Kugel (10) und Öffnungen (11) in der Seitenwandung (1') des röhrenförmigen Metallteils (1), wobei die Kugel (10) zwischen einer den Durchgang zur trichterförmigen Aufweitung (2) freigebenden und einer diesen verschließenden Stellung bewegbar ist.

9. Prothese nach Anspruch 8, bei der die Öffnungen (11) in der Seitenwandung (1') durch Schutzlaschen (13) vor dem Eintreten von Fremdkörpern in die Trachea geschützt sind.

10. Stimmprothese nach Anspruch 7, bei der das Ventil ein Flatterventil ist, gebildet aus einem über das röhrenförmige Metallteil (1) geschobenen Kunststoffschlauch (14), welcher am oesophagusseitigen Ende bis auf einen Längsschlitz (15) verschlossen ist.

11. Stimmprothese nach Anspruch 6, bei der das röhrenförmige Metallteil (1) oesophagusseitig an seiner Stirnöffnung verschlossen ist und in diesem Bereich in der Seitenwandung (1') mindestens eine Seitenöffnung (16) aufweist, welche durch eine schützende, den aus der Seitenöffnung (16) austretenden Luftstrom umleitende Abdeckhaube (17) überdeckt ist.

12. Stimmprothese nach Anspruch 6, bei der zusätzlich tracheaseitig eine Schutzvorrichtung in Form eines Einwege-Kugelventils (18) vorgesehen ist.

13. Stimmprothese nach Anspruch 12, bei der im Inneren des röhrenförmigen Metallteils (1) im tracheaseitigen Bereich eine Kugel (19) freibeweglich gelagert ist, die in ihrer Bewegung in Richtung fort von der trichterförmigen Aufweitung (2) begrenzt ist durch einen das röhrenförmige Metallteil (1) diametral durchsetzenden Anschlag (20) und in ihrer Bewegung in Richtung der trichterförmigen Aufweitung begrenzt ist durch einen in diesem ausgeformten Kugelventilsitz (21).

14. Stimmprothese nach Anspruch 12, bei der die tracheaseitige Schutzvorrichtung in einem länglichen, röhrenförmigen Aufsatzstück (22 ) ausgebildet ist, welches auf die trichterförmige Aufweitung aufgeschoben ist, die trichterförmige Aufweitung (2) hintergreift und in seinem Inneren die Kugel (24 ) des Einwege-Kugelventils (18) freibeweglich lagert, die in ihrer Bewegung in Richtung auf die trichterförmige Aufweitung (2) durch einen ihr Lumen diametral durchsetzenden Anschlag (25) und in ihrer Bewegung in Richtung auf die Lufteintrittsöffnung (26) durch einen in ihrem Inneren ausgebildeten Kugelventilsitz (27) begrenzt ist.

## Claims

1. Voice prosthesis for use in the shunt between the trachea and oesophagus of a patient having undergone a laryngectomy, consisting of a tubular metal part (1) with an open funnel-shaped widening (2) on the trachea side, characterised by the fact that inside the air flow passage of the prosthesis from the trachea to the oesophagus, there is at least one element (3) which can be made to vibrate by the air flow passing through it and thus produce an audible sound.

2. Voice prosthesis per claim 1, in which the sound-producing element is a flexible membrane (4, 4', 4'', 4''') which covers the funnel-shaped widening (2), and which reaches behind the edge (5) of the funnel-shaped widening (2) and has at least one slit (6, 6', 6'', 6''') in the area of the widening (2) it covers.

3. Voice prosthesis per claim 1, in which the funnel-shaped widening (2) is designed such that the membrane (4, 4', 4'', 4''') does not touch the base (7) of the widening (2) even at maximum vibration amplitude.

4. Voice prosthesis per claim 2 or 3 in which the membrane (4, 4', 4'', 4''') is made of silicone rubber with a Shore hardness in the range 30 to 70.

5. Voice prosthesis per claim 1, in which the sound-producing element is a metal voice platelet (8) anchored in the wall of the free lumen of the tubular metal part (1).

6. Voice prosthesis per one of the claims 1 to 5, which is protected at least on the oesophagus side by a safety device (9) so that particles of food and saliva cannot enter.

7. Voice prosthesis per claim 6, in which the tubular metal part (1) can be closed on the oesophagus side by a one-way valve (9', 9'').

8. Voice prosthesis per claim 7, in which the valve is formed by a ball (10) which can move inside the tubular metal part (1) and apertures (11) in the side wall (1') of the tubular metal part (1), whereby the ball (10) can move between a position which clears the passage to the funnel-shaped widening (2) and a position which closes this.

9. Prosthesis per claim 8, in which the apertures (11) in the side wall (1') are protected by safety flaps (13) to prevent foreign bodies entering the trachea.

10. Voice prosthesis per claim 7, in which the valve is a float valve made from a plastic tube (14) pushed over the tubular metal part (1), and which is closed at the end on the oesophagus side down to an elongated slit (15).

11. Voice prosthesis per claim 6, in which the front aperture in the tubular metal part (1) is sealed on the oesophagus side and has at least one side aperture (16) in this area in the side wall (1'), this side aperture (16) being covered by a protective cowling (17) which diverts the flow of air coming from the side aperture (16).

12. Voice prosthesis per claim 6, in which additionally on the trachea side there is a safety device in the form of a one-way ball valve (18).

13. Voice prosthesis per claim 12, in which a ball is mounted so that it can move freely inside the tubular metal part (1) in the area on the trachea side, the movement of this ball being restricted in the direction away from the funnel-shaped widening (2) by a stop (20) passing diametrally through the tubular metal part (1) and in the direction of the funnel-shaped widening by a ball valve seat (21) formed in it.

14. Voice prosthesis per claim 12, in which the safety device on the trachea side is in the form of an elongated tubular cap (22) which is pushed over the funnel-shaped widening, reaches behind the funnel-shaped widening (2) and in which the ball (24) of the one-way ball valve (18) is mounted, the movement of the ball being restricted in the direction of the funnel-shaped widening (2) by a stop (25) passing diametrally through its lumen and in the direction of the air inlet aperture (26) by a ball valve seat (27) formed inside it.

## Revendications

1. Prothèse vocale à placer dans la dérivation entre la trachée et l'oesophage d'un patient laryngectomisé, se composant d'une pièce métallique en forme de conduit (1) muni d'un évasement en forme d'entonnoir ouvert (2) du côté trachéal, caractérisée en ce que, dans le trajet du courant d'air de la prothèse provenant de la trachée vers l'oesophage, est installé au moins un élément (3) qui peut être déplacé en oscillation par l'écoulement d'air entrant par la prothèse et qui produit de cette façon un son audible.

2. Prothèse vocale selon la revendication 1, caractérisée en ce que l'élément produisant le son est une membrane flexible (4, 4', 4'', 4''') recouvrant l'évasement en forme d'entonnoir (2), qui se resserre derrière le bord (5) de l'évasement en forme d'entonnoir (2) et qui comporte au moins une fente (6, 6', 6'', 6''') dans la région de l'évasement (2) recouvert par elle.

3. Prothèse vocale selon la revendication 1, caractérisée en ce que l'évasement en forme d'entonnoir (2) est dimensionné de façon que la membrane (4, 4', 4'', 4''') n'atteigne pas le fond de l'évasement (2), même lors de l'amplitude d'oscillation maximale.

4. Prothèse vocale selon la revendication 2 ou 3, caractérisée en ce que la membrane (4, 4', 4'', 4''') se compose de caoutchouc au silicone ayant une dureté Shore de l'ordre de 30 à 70.

5. Prothèse vocale selon la revendication 1, caractérisée en ce que l'élément produisant le son est une lamelle vocale métallique (8) placée dans l'espace libre de la pièce métallique en forme de conduit (1) et ancrée dans la paroi (1') de celle-ci.

6. Prothèse vocale selon l'une quelconque des revendications 1 à 5, caractérisée en ce qu'elle est protégée au moins du côté oesophage de l'entrée de particules digestives et de salive par un dispositif de protection (9).

7. Prothèse vocale selon la revendication 6, caractérisée en ce que la pièce métallique en forme de conduit (1) peut être fermée côté oesophage par une valve anti-retour (9', 9'').

8. Prothèse vocale selon la revendication 7, caractérisée en ce que la valve est formée d'une bille (10) qui se déplace à l'intérieur de la pièce métallique en forme de conduit (1) et d'ouvertures (11) dans la paroi latérale (1') de la pièce métallique en forme de conduit (1), la bille (10) pouvant se déplacer entre une position qui débloque le passage vers l'évasement en forme d'entonnoir (2) et une position qui ferme celui-ci.

9. Prothèse vocale selon la revendication 8, caractérisée en ce que dans la paroi latérale (1') les ouvertures (11) sont protégées de l'entrée de corps étrangers dans la trachée par des languettes de protection (13).

10. Prothèse vocale selon la revendication 7, caractérisée en ce que la valve est une valve flottante, formée d'un tuyau en matière plastique (1) glissé sur la pièce métallique en forme de conduit (1), qui est fermé à l'extrémité du côté de l'oesophage à l'exception d'une fente longitudinale (15).

11. Prothèse vocale selon la revendication 6, caractérisée en ce que la pièce métallique en forme de conduit (14) du côté oesophage est fermée au niveau de son ouverture frontale et présente au moins une ouverture latérale (16) dans cette région dans la paroi latérale (1'), qui est recouverte d'un capuchon (17) de protection, déviant l'écoulement d'air sortant par l'ouverture latérale (16).

12. Prothèse vocale selon la revendication 6, caractérisée en ce qu'en plus du côté trachée est prévu un dispositif de protection sous la forme d'une valve à bille anti-retour (18).

13. Prothèse vocale selon la revendication 12, caractérisée en ce qu'à l'intérieur de la pièce métallique en forme de conduit (1) dans la région du côté de la trachée est logée libre en déplacement une bille (19), qui est limitée dans son déplacement dans la direction opposée à l'évasement en forme d'entonnoir (2) par une butée (20) traversant diamétralement la pièce métallique en forme de conduit (1) et qui est limitée dans son déplacement en direction de l'évasement en forme d'entonnoir par un siège de valve à bille (21) formé dans celui-ci.

14. Prothèse vocale selon la revendication 12, caractérisée en ce que le dispositif de protection du côté de la trachée est développé selon une pièce rapportée en forme de conduit, allongée (22), qui est glissée sur l'évasement en forme d'entonnoir, qui se resserre derrière l'évasement en forme d'entonnoir (2) et qui loge en déplacement libre en son centre la bille (24) de la valve à bille anti-retour (18), qui est limitée dans son déplacement en direction de l'évasement en forme d'entonnoir (2) par une butée (25) traversant diamétralement son espace vide et dans son déplacement en direction de l'entrée d'admission d'air (26) par un siège de valve à bille (27) développé en son centre.
